# EUROPÄISCHE PATENTANMELDUNG

(11) **EP 2 402 456 A1**
(43) Veröffentlichungstag der Anmeldung: **04.01.2012**
(21) Anmeldenummer: 10167867.0
(22) Anmeldetag: 30.06.2010
(51) Int. Cl.: C12Q 1/68

(54) **Verfahren zur Ermittlung von Organismen in Wasser**

(71) Anmelder: Imeth AG, 8621 Witzikon (CH)
(72) Erfinder: Wohler, Christian, 5612, Villmergen (CH); Krapf-Kovalj, Tamara, 8942, Oberrieden (CH); Gantenbein-Demarchi, Corinne, 8134, Adliswil (CH); Kuhn, Roger, 8910, Affoltern am Albis (CH)
(74) Vertreter: Hepp Wenger Ryffel AG

(57) **Zusammenfassung**

Die vorliegende Erfindung betrifft ein Verfahren zur Ermittlung von Organismen in Wasser, insbesondere in Trinkwasser. Eine Wasserprobe wird über einen Filter filtriert und Organismenzellen werden mit einem Lösungsmittel aus dem Filter gewonnen. Die Organismenzellen werden lysiert und anschliessend bestimmt und/oder quantifiziert. Insbesondere hat sich eine Quantifizierung und/oder Bestimmung mittels RT-qPCR als geeignet erwiesen.

## Beschreibung

Die vorliegende Erfindung betrifft ein Set und ein Verfahren zur Ermittlung von Organismen im Wasser, insbesondere Trinkwasser, gemäss den Oberbegriffen der unabhängigen Ansprüche.

Unsauberes und kontaminiertes Trinkwasser, sowie mangelhaft aufbereitetes Abwasser sind weltweit eine der Hauptursachen vermeidbarer Krankheits- und Todesfälle. Es wird angenommen, dass Durchfallerkrankungen im Zusammenhang mit verunreinigtem Wasser alleine zwischen 2.2 und 3.4 Mio Todesfälle pro Jahr verursachen. Zugang zu sicherem Trinkwasser ist essentiell für die Erhaltung der menschlichen Gesundheit.

Neben chemischen Verunreinigungen spielt die mikrobiologische Sicherheit von Trinkwasser eine entscheidende Rolle in der Prävention von Krankheiten. Das grösste mikrobiologische Risiko besteht bei der Einnahme von Wasser, das mit menschlichen oder tierischen Fäkalien kontaminiert ist. Fäkalien können die Quelle pathogener Bakterien, Viren, Protozoen und Würmer sein.

Viele dieser Krankheitserreger können nicht oder nur mit beträchtlichem Aufwand spezifisch nachgewiesen werden. Insbesondere ist es aufwendig, Viren und Parasiten (z.B. *Cryptosporidium* und Giardia) nachzuweisen.

In der Praxis hat sich der Nachweis von so genannten Indikatororganismen bewährt. Der Nachweis eines Indikatororganismus weist auf die mögliche Anwesenheit ähnlicher pathogener Organismen hin. Zudem lässt sich aus der Anwesenheit eines Indikatororganismus schliessen, dass im Verlauf der Wasseraufbereitung ein Fehler unterlaufen ist.

Obwohl der Schluss, dass bei Abwesenheit des Indikators das Wasser grundsätzlich frei von pathogenen Keimen ist, nicht allgemein gültig ist, so ist deren Anwesenheit doch ein sehr nützlicher und beinahe sicherer Faktor in der Bestimmung kontaminierten Wassers. In der Vergangenheit hat sich der Nachweis von insbesondere coliformen Keimen bewährt. Der positive Nachweis coliformer Keime ist ein guter Hinweis für das Vorhandensein fäkaler Kontamination. Die WHO legt Richtlinien und Empfehlungen fest, wie und in welchem Ausmass coliforme Keime zur Bestimmung der Trinkwassersicherheit herangezogen werden können (Guidelines for drinking water quality, 3rd edition, Vol.1, Table 7.7, Genf 2008). So sollen beispielsweise in einer 100 ml Probe Trinkwasser keine *E. coli* oder thermotolerante coliforme Keime nachzuweisen sein.

Diese Nulltoleranz für coliforme Keime für Trinkwasser für den menschlichen Gebrauch ist in vielen Ländern gesetzlich verordnet (National Primary Drinking Water Regulations, US Environmental Protection Agency, 2009; EU Richtlinie 98/89/EG; Schweizer Hygieneverordnung vom 23.November 2005).

Zum Nachweis von *E. coli* existieren standardisierte Verfahren. Üblicherweise wird eine entsprechend Wasserprobe über eine Membran filtriert und die Keime werden anschliessend auf Agar inkubiert. Aerobe mesophile Keime können zudem direkt als Gusskultur inkubiert werden. Mit diesem Verfahren lässt sich auch eine quantitative Bestimmung der Keimzahl erreichen. Nachteilig an diesem Standardverfahren ist jedoch die vergleichsweise lange Inkubationszeit von mindestens 24 Stunden bis hin zu 3 Tagen. Es versteht sich von selbst, dass eine derart lange Frist bis zum endgültigen Bescheid zur Trinkwassersicherheit erhebliche Risiken birgt.

Moderne molekularbiologische Verfahren gestatten mittlerweile auch eine quantitative Bestimmung von Indikatorkeimen aus Trinkwasser. Dennoch kommen auch diese Verfahren nicht ohne einen Aufarbeitungs- oder Inkubationsschritt aus.

US 2007/0196884 A1, zum Beispiel, zeigt ein Verfahren zum Nachweis von coliformen Keimen, insbesondere von *E. coli* in Trinkwasser. Das Verfahren verwendet einen enzymatischen Nachweis. Selektive Enzyme (β-glucoronidase und β-galactosidase) werden mit einer Induktionslösung induziert. Die Probe wird bis zu zwei Stunden inkubiert und der enzymatische Nachweis fluorimetisch durchgeführt. Nachteilig an dieser Methode ist, dass sie keine Rückschlüsse auf die Keimzahl ermöglicht.

Es ist somit eine Aufgabe der vorliegenden Erfindung, eine Lösung für die Nachteile des Stands der Technik zu bieten. Insbesondere soll ein Verfahren und ein Set zur Verfügung gestellt werden, das einen raschen, kostengünstigen, einfach zu bedienenden und ohne beträchtliche Zeitverzögerung einsetzbaren Nachweis für Organismen in Trinkwasser ermöglicht.

Die Nachteile des Standes der Technik werden mit einem Verfahren und einem Set gemäss kennzeichnendem Teil der unabhängigen Ansprüche gelöst.

Ein Aspekt der vorliegenden Erfindung betrifft also ein Verfahren zur Ermittlung von Organismen in Wasser, insbesondere in Trinkwasser. Eine Wasserprobe wird über einen Filter filtriert und Organismenzellen werden mit einem Lösungsmittel aus dem Filter gewonnen. Die Organismenzellen werden lysiert und anschliessend bestimmt und/oder quantifiziert. Insbesondere hat sich eine Quantifizierung und/oder Bestimmung mittels RT-qPCR als geeignet erwiesen. Überraschend wurde gefunden, dass der Filter im Wesentlichen mit dem Lösungsmittel aufgelöst werden kann und dabei das Lösungsmittel die Organismenzellen im Wesentlichen intakt lässt.

Die zu ermittelnden Organismen-Zellen können alle mikrobiellen Keime sein, die Rückschlüsse auf das Vorhandensein von Keimen im Wasser ermöglichen. Als besonders geeignet haben sich coliforme Bakterien herausgestellt. Im Sinne der vorliegenden Anmeldung werden hierzu insbesondere die Geni *Escherichia, Citrobakter, Enterobakter* und *Klebsiella* gezählt. Auch andere Enterobakterien oder pathogene Bakterien, wie z.B. *Salmonella, Yersinia, Legio*nella und andere können mit dem vorliegenden Verfahren nachgewiesen werden.

In der vorliegenden Anmeldung bezieht sich RT-qPCR auf "real *time quantitative PCR";* die RT-qPCR basiert auf dem Prinzip der an sich hinlänglich bekannten herkömmlichen Polymerase Kettenreaktion (PCR). Sie gestattet zusätzlich die Quantifizierung der gewonnenen Nukleinsäure-Fragmente. Mit Hilfe von Fluoreszenz-Messungen (vorzugsweise unter Verwendung von TaqMan® Sonden, auch Hydrolyse-Sonden genannt) wird während der PCR-Zyklen proportional die Menge der PCR Produkte ermittelt. Der RT-qPCR vorgelagert ist im Rahmen der Erfindung vorzugsweise eine reverse Transkription.

Auch Verfahren zum Lysieren von Zellen sind in der biologischen Forschung und Anwendung seit längerem bekannt. Für die vorliegende Erfindung sind sowohl mechanische, chemische als auch enzymatisch/biologische Lyseverfahren geeignet.

In einer bevorzugten Ausführungsform wird der Filter mit einem polaren aprotischem Lösungsmittel im Wesentlichen aufgelöst.

Ein polar aprotisches Lösungsmittel verfügt über polare funktionelle Gruppen und weist ein Dipolmoment auf. Verschiedene polare aprotische Lösungsmittel sind dem Fachmann bekannt. Unter anderem existieren polar aprotische Lösungsmittel aus den Gruppen der Ketone, Laktone, Nitrile, Nitro-Verbindungen, tertiäre Carbonsäureamide, Harnstoffderivate, Sulfoxide, Sulfone und Kohlensäure-Ester. Besonders bevorzugt wird das Lösungsmittel und das Filtermaterial so aufeinander abgestimmt ausgewählt, dass es zu einer Auflösung des Filters kommt. Überraschend wurde gefunden, dass wenn das aprotische Lösungsmittel so ausgewählt wird, dass es gerade entgegen der Empfehlung des Herstellers den Filter auflöst, die Organismenzellen dennoch weitgehend intakt aus dem Lösungsmittel gewonnen werden können.

In einer bevorzugten Ausführungsform wird der Filter mit Dimethylsulfoxid (DMSO) im Wesentlichen aufgelöst und die Organismen werden im Wesentlichen intakt aus dem Dimethylsulfoxid gewonnen.

In einer weiteren besonderen Ausführungsform wird ein Celluloseester-Filter verwendet und mit einem polaren aprotischen Lösungsmittel im Wesentlichen aufgelöst und die Organismenzellen im Wesentlichen intakt aus den polaren aprotischen Lösungsmittel gewonnen. Bevorzugt wird hierbei Dimethylsulfoxid als polares aprotisches Lösungsmittel verwendet.

In einer weiteren bevorzugten Ausführungsform wird Celluloseacetat als Filtermaterial verwendet.

Ein weiterer Aspekt der vorliegenden Erfindung betrifft ein Set zur Ermittlung von Organismen im Wasser, insbesondere Trinkwasser. Das Set enthält mindestens ein Lösungsmittel und einen Filter. Mindestens ein Lösungsmittel des Sets ist in der Lage, den Filter im Wesentlichen aufzulösen ohne die Organismenzellen im Wesentlichen zu schädigen.

In einer weiteren bevorzugten Ausführungsform enthält das Set zudem eine Spritze zur Entnahme einer Wasserprobe des zu untersuchenden Wassers. Vorzugsweise ist die Spritze bereits auf die standardmässig zu untersuchende Trinkwassermenge normiert, insbesondere auf 50 ml oder 100 ml.

In einer besonders bevorzugten Ausführungsform besteht der Filter im Wesentlichen aus mindestens einem Celluloseester und das Lösungsmittel umfasst mindestens ein polares aprotisches Lösungsmittel.

Bevorzugt wird das Lösungsmittel ausgewählt aus der Gruppe bestehend aus: Ketonen, Lactonen, Nitrilen, Nitro-Verbindungen, tertiären Carbonsäureamiden, Harnstoffderivaten, Sulfoxiden, Sulfonen und Kohlensäure-Ester. Besonders bevorzugt wird das Lösungsmittel ausgewählt aus der Gruppe bestehend aus: Aceton, 4-Butyrolacton, Acetonitril, Nitromethan, Dimethylformamid, Tetramethyl Harnstoff, Dimethylpropylen-Harnstoff, Sulfolan, Dimethyl Carbonat, Ethylen Carbonat und Dimethylsulfoxid.

In einer bevorzugten Ausführungsform besteht der Filter im Wesentlichen aus Celluloseacetat.

Insbesondere vorteilhaft ist die Kombination aus einem Filter, der im Wesentlichen aus Celluloseacetat besteht und einem Lösungsmittel, das Dimethylsulfoxid ist oder im wesentlichen umfasst. Obschon von Dimethylsulfoxid zur Verwendung mit Filtern auf Celluloseacetat-Basis im Stand der Technik abgeraten wird, wurde überraschend und entgegen der gängigen Lehre gefunden, dass sich der Filter im Rahmen der Erfindung gerade vorteilhaft auflösen lässt und die Organismenzellen im Wesentlichen unbeschädigt bleiben.

Das Set kann weiterhin Pufferlösungen und Adjuvantien enthalten, welche die Handhabung und die Reaktionen zur Ermittlung von Organismen im Wasser ermöglichen und erleichtern. Solche Pufferlösungen und Adjuvantien sind dem Fachmann bekannt.

In einer weiteren bevorzugten Ausführungsform beinhaltet das Set zudem mindestens ein PCR Primerpaar, insbesondere ein Primerpaar für RT-qPCR, welches geeignet ist, unter stringenten Bedingungen an die DNA oder RNA aus Keimen aus einer Wasserprobe zu hybridisieren. Vorzugsweise enthält das Set zusätzlich eine Sonde zur Ermöglichung der quantitativen Bestimmung der PCR Produkte, besonders bevorzugt mittels Fluoreszenzanalyse; eine derartige Sonde vermag an die zu detektierende DNA oder RNA unter stringenten Bedingungen zu hybridisieren und verfügt an ihrem einen Ende über einen Quencher, an ihrem anderen Ende über einen Reporter-Fluoreszenzfarbstoff.

Besonders bevorzugt enthält das Set zur Bestimmung von *Escherichia coli* das Primerpaar Seq ID Nr. 2 und Seq ID Nr. 3; und/oder das Primerpaar Seq ID Nr. 2 und Seq ID Nr. 4. Die vorgenannten Sets enthalten vorzugsweise zusätzlich jeweils die Sonde Seq ID Nr. 1, die mit einem geeigneten Fluoreszenzquencher (vorzugsweise BHQ1 oder TAMRA) an ihrem einen Ende (vorzugsweise 3') und mit einem geeigneten Fluoreszenzfarbstoff (vorzugsweise FAM oder Yakima yellow) an ihrem anderen Ende (vorzugsweise 5') modifiziert ist.

Weiter besonders bevorzugt enthält das Set zur Bestimmung von *Enterococcus faecalis* das Primerpaar Seq ID Nr. 6 und Seq ID Nr. 7. Das vorgenannte Set enthält vorzugsweise zusätzlich die Sonde Seq ID Nr. 5, die mit einem geeigneten Fluoreszenzquencher (vorzugsweise BHQ1 oder TAMRA) an ihrem einen Ende (vorzugsweise 3') und mit einem geeigneten Fluoreszenzfarbstoff (vorzugsweise FAM oder Yakima yellow) an ihrem anderen Ende (vorzugsweise 5') modifiziert ist.

Neben den identischen in dieser Patentanmeldung beschriebenen Sequenzen sind selbstverständlich auch abgewandelte Sequenzen im Rahmen der Erfindung anwendbar, sofern sie unter gleich stringenten Bedingungen vergleichbar gut mit einer zu den hierin genannten Sequenzen komplementären Sequenz zu hybridisieren vermögen, wobei diese komplementären Sequenzen zu ≥ 90% mit den hierin genannten Sequenzen überlappen; und/oder ≥ 90% Sequenzhomologie zu den hierin genannten Sequenzen aufweisen.

Im Folgenden wird die vorliegende Erfindung anhand eines konkreten Praxisbeispiels weiter erläutert, ohne dass die Erfindung hierauf zu beschränken ist.

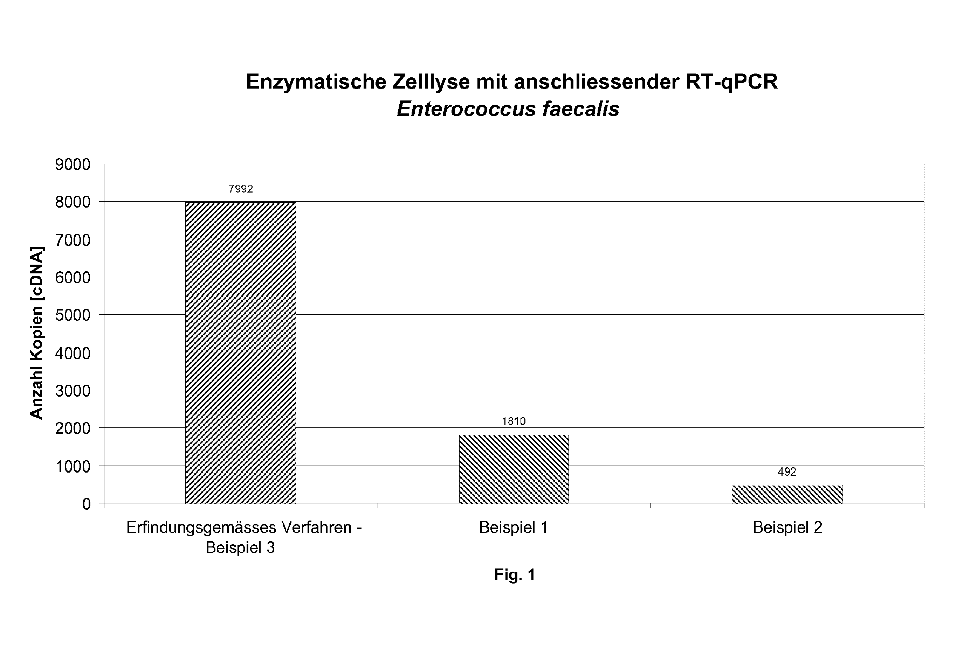

**Fig. 1** zeigt eine Gegenüberstellung von Resultaten des erfindungsgemässen Verfahrens mit Vergleichsversuchen gemäss gängigen Verfahren.

### Vergleichsbeispiel 1:

In diesem Vergleichsversuch wurde *Enterococcus faecalis* in 1 ml H₂O nachgewiesen. Gemessen wurde die Anzahl Kopien des Zielmoleküls (16S rRNA) .

Die Bakterien wurden über Nacht in einem Schüttler bei 37°C in BHI Bouillon aufgezogen. Die Kulturen wurden anschliessend in zwei 50 ml Eppendorf Tubes alliquotiert und zentrifugiert / pelletiert (4 °C, 5'000 g, 5 min). Anschliessend wird der Überstand verworfen und das Pellet mit je 10 mL 0.9 % NaCl resuspendiert und wieder für 5 Min. bei 4 °C und 5000 g zentrifugiert/pelletiert. Dieser Schritt wird 2 Mal wiederholt. Das Pellet wird in je 5 mL 0.9 % NaCl resuspendiert und zusammengeführt. Die Gesamtkeimzahl wird mit Hilfe einer Zählkammer bestimmt und eine Verdünnungsreihe von 10⁹ - 10° Zellen/mL erstellt. Zwecks Ermittlung der Lebendkeimzahl wird parallel ein kultureller Ansatz auf PC-Agarplatten erstellt. Je Verdünnungsschritt wird 1 mL der Bakteriensuspension in ein 2 mL RNase free Eppendorf tube pipettiert und während 15 Min. bei 4°C und 13'200 rpm zentrifugiert. Der Überstand wird verworfen und die enzymatische Lyse der Bakterien erfolgt entweder, wie unten im Detail aufgeführt, mit dem RNeasy Mini Kit (Qiagen, Inc.), Eluat 30 µl oder dem RNeasy MiniElute Kit (Qiagen, Inc.), Eluat 10-12 µl.

### Vergleichsbeispiel 2:

In diesem Vergleichsversuch wurde *Enterococcus faecalis* in 1 ml H₂O nachgewiesen. Gemessen wurde die Anzahl Kopien des Zielmoleküls (16S rRNA) .

Je Verdünnungsschritt wird 1 mL der Bakteriensuspension mit einer 5 ml Spritze aufgezogen und durch einen Spritzenvorsatzfilter 0.45 µm filtriert. Anschliessend wird eine zweite, an den Filter von der anderen Seite gekoppelte Spritze mit 2 mL DEPC behandeltem Wasser befüllt und die auf dem Filter befindlichen Zellen zurück in die erste Spritze gespült. Die 2 mL Wasser werden in ein 2 mL RNase free eppendorf tube gegeben und während 15 Min. bei 4°C und 13'200 rpm zentrifugiert. Der Überstand wird verworfen und die enzymatische Lyse erfolgt entweder, wie unten aufgeführt, mit dem RNeasy Mini Kit, Eluat 30 µl oder dem RNeasy MiniElute Kit (Qiagen Inc.), Eluat 10-12 µl.

### Beispiel 3 (erfindungsgemäss):

In diesem erfindungsgemässen Versuch wurde *Enterococcus faecalis* in 1 ml H₂O nachgewiesen. Als Filtermedium wurde Minisart (17598, 0,45 µm, Celluloseacetat) von Sartorius Dedin Biotech S.A. (Aubagne Cedex, France) verwendet. Als Lösungsmittel wurde Dimethylsulfoxid (Fluka: 41638) verwendet.

Die Wasserprobe wird mit einer Spritze (z.B. 100 mL, Huber) aufgezogen. Die Spritze wird mit dem Filter verschraubt und anschliessend wird die Probe langsam durch den Filter gepresst. Mit Hilfe einer Spritze und Kanüle werden 2 ml DMSO aufgezogen. Die Spritze wird mit dem Filter verschraubt und auf ein 2 ml Safe-Lock Eppendorff Biopur (Katalog Nr. 0030121.597) gesetzt.

DMSO wird bis zum Druckpunkt in das Filtergehäuse gepresst. Nach 5-10 Sekunden Einwirkzeit beginnt sich das Celluloseacetat aufzulösen.

Das restliche DMSO wird durch das Filtergehäuse in das Eppendorff Tube gepresst.

Das Eppendorff Tube wird 15 Minuten bei 13'200 RPM (20° bis 25° C.) zentrifugiert. Der Überstand wird verworfen und das Tube mit der Öffnung nach unten auf ein Papiertuch gesetzt. Nach 10-20 Sekunden Trocknung wird der Deckel geschlossen und man beginnt mit der enzymatischen Lyse. Verfahren zur Lyse von Bakterien sind dem Fachmann einschlägig bekannt. Für die Lyse bestehen fertige Komponenten Kits zur Verfügung. Im vorliegenden Beispiel wurde "RNeasy mini kit" von Qiagen, Inc. gemäss Protokoll des Vertreibers verwendet.

Es wurden RNase freie Reagenzien verwendet. Lysepuffer: TE Puffer (10 mM Tris•Cl, 1 mM EDTA, pH 8.0) mit 20 mg/ml Lysozym. Salm Sperm DNA 10 mg/ml (Sigma, 31149). RLT Puffer: 10 µl Beta-Mercaptoethanol per 1 ml Puffer RLT (Quiagen, Inc.) wird hinzugefügt und gemischt.

Das entsprechende Volumen Salm Sperm DNA (10 µg) and TE buffer mit Lysozym wird hinzugefügt (gemäss Tabelle).

| Number of bacteria | RNeasy spin column | Salm Sperm DNA [0 mg/ml (Step 3) | TE buffer containing lysozyme (step 3) | Buffer RLT (step 5) | Ethanol (100%) (step 6) |
|---|---|---|---|---|---|
| <5 × 10⁸ | Mini | 1 µl | 100 µl | 350 µl | 250 µl |

Anschliesend wird im Vortex für 10 sek gemischt und bei Raumtemperatur für 5 min in einem Schüttel Inkubator inkubiert. Das entsprechende Volumen RLT Puffer (gemäss Tabelle) wird hinzugefügt. Sofern Partikel sichtbar sind, zentrifugieren, pelletieren und den Überstand weiterverwenden. Das entsprechende Volumen Ethanol wird hinzugefügt (gemäss Tabelle) und mit der Pipette gemischt.

Die Probe (700 µl) wird auf eine RNeasy mini Säule transferiert und auf ein 2 ml Tube gesetzt. Zentrifugieren: 15 s bei > 8000 x g (>10,000 rpm). Durchfluss verwerfen und die Säule weiterverwenden.

Die RNeasy mini Säule wird auf ein neues 2 ml Tube platziert. 700 µl Puffer RW1 (Quiagen, Inc.) wird auf die Säule aufgetragen. Zentrifugieren: 15 s at >8000 x g (>10,000 rpm) um die Säule zu waschen. Den Durchfluss verwerfen und Säule weiterverwenden.

500 µl Puffer RPE (Quiagen, Inc.) auf die RNeasy mini Säule auftragen. Zentrifugieren: 15 Sekunden bei > 8000 x g (> 10'000 rpm) um die Säule zu waschen, anschliessend den Durchfluss verwerfen und die Säule weiterverwenden.

500 µl Puffer RPE (Quiagen, Inc.) auf die RNeasy mini Säule auftragen. Zentrifugieren: 2 Minuten bei > 8000 x g (> 10'000 rpm) um die Säule zu waschen, anschliessend den Durchfluss verwerfen und die Säule weiterverwenden.

Die RNeasy mini Säule wird auf ein neues 2 ml Tube platziert und für 1 Minute bei voller Kraft zentrifugiert. Den Durchfluss verwerfen und Säule weiterverwenden.

Die RNeasy mini Säule wird auf ein neues 1.5 ml Tube (RNAsefrei) platziert. 30 µl RNase-free Wasser wird direkt auf die Mitte der Säulenmembran aufgetragen. Zentrifugieren: 1 min bei voller Kraft, um die RNA zu eluieren.

Für die reverse Transkription wurde der First Strand cDNA Synthesis Kit for RT-PCR (AMV) (Roche, Cat. No. 11 483 188 001) verwendet. Abweichend vom Herstellerprotokoll wurde RNA von Bakteriophage MS2 (10 µg/ml) (Roche, Cat. No. 10 165 948 001) oder baker's yeast tRNA (10 µg/ml) (Roche, Cat. No. 10 109 495 001) eingesetzt. Im Falle von Escherichia coli wurde RNasin® Ribonuclease Inhibitor (Promega N211) nach Angaben des Herstellers eingesetzt.

Temperatur-Zeit-Profil für die reverse Transkription: 1 Zyklus, 25°C 10 Min.; 42°C 10 Min.; 99°C 5 Min.

Für die RT-qPCR wurde ein LightCycler® 480 Probes Master (Roche 4707494001) verwendet.

RT-qPCR Temperatur-Zeit-Profil:
Pre-Incubation 1 Zyklus, 7 Min., 95 °C
Amplification 45 Zyklen
   Denaturation 10 sek., 95 °C
   Annealing 30 sek., 53°C
   Elongation 1 sek., 72 °C

Die folgenden Primer/Sonden wurden verwendet:

### A. Escherichia coli

*Escherichia coli* Primer/Sonden:

| | **Name** | **bp** | **Tₘ°C** | **G/C %** |
|---|---|---|---|---|
| SEQ. ID No. 1 | Coli2Probe_b (Sonde) | 29 | 64 | 52 |
| SEQ. ID No. 2 | Coli fw_2b (forward Primer) | 20 | 54 | 60 |
| SEQ. ID No. 3 | Coli2d_re (reverse Primer) | 22 | 50 | 50 |
| SEQ. ID No. 4 | Coli2e_re (reverse Primer) | 22 | 50 | 50 |

Sondenmodifikation: FAM - BHQ1

### B. Enterococcus faecalis

*Enterococcus faecalis* Primer/Sonden:

| | **Name** | **bp** | **Tₘ°C** | **G/C %** |
|---|---|---|---|---|
| SEQ. ID No. 5 | Efaec16Sprobe_1 (Sonde) | 27 | 62 | 52 |
| SEQ. ID No. 6 | Efaec16s fw_1 (forward Primer) | 20 | 54 | 55 |
| SEQ. ID No. 7 | Efaec16S re_1 (reverse Primer) | 25 | 54 | 40 |

Sondenmodifikation: Yakima yellow - BHQ1

### Ergebnisse:

Fig.1 fasst die Ergebnisse zusammen. Das Ergebnis des erfindungsgemässen Beispiels 3 ergibt eine Anzahl von 7992 cDNA Kopien, wohingegen die alternativen Verfahren, wie dem Zentrifgieren/Pelletieren, ohne Isolation über einen Filter (Vergleichsbeispiel 1) und das Isolieren der Zellen mittels Rückspülen des Filters (Vergleichsbeispiel 2) eine Anzahl von lediglich 1810, respektive 492 Kopien cDNA ergeben.

Mit dem erfindungsgemässen Verfahren wird somit eine um ein Vielfaches besser Wiederfindung und damit verbunden eine tiefere Nachweisegrenze erreicht, als mit den gängigen Verfahren.

Weitere Primer und Sonden wurden getestet und für den Einsatz im Rahmen der Erfindung als tauglich befunden (fw bezeichnet den forward primer; re bezeichnet den reverse primer; probe bezeichnet die Sonde; die Sonden können, wie vorstehend beschrieben, modifiziert sein mit Qencher(n) und Fluoreszenzfarbstoff(en)):

### C. Escherichia coli

### Locus 1 (16S)

| | |
|---|---|
| SEQ. ID No. 8: | 618 E.coli 16S fw |
| SEQ. ID No. 9: | Ecoli16S_1 fw |
| SEQ. ID No. 10: | 606 Ecoli 16S fw |
| | |
| SEQ. ID No. 11: | 698 E.coli 16S re |
| SEQ. ID No. 12: | 685 Ecoli 16S re |
| SEQ. ID No. 13: | 643 E.coli16S probe |

### Locus 2 (16S)

| | |
|---|---|
| SEQ. ID No. 14: | 443 Ecoli16Sprobe |
| SEQ. ID No. 15: | 404 Ecoli 16S fw |
| SEQ. ID No. 16: | 481 Ecoli 16S re |
| | |
| SEQ. ID No. 17: | Wolc Probe |
| SEQ. ID No. 18: | Wolc fw |
| SEQ. ID No. 19: | Wolc re |
| | |
| SEQ. ID No. 20: | Ecoli16sProbe_2 |
| SEQ. ID No. 21: | Coli fw_2.2 |
| SEQ. ID No. 22: | 404 Ecoli 16S fw |
| SEQ. ID No. 23: | 481 Ecoli 16S re |

### Locus 3 (16S)

| | |
|---|---|
| SEQ. ID No. 24: | Coli fw_2b |
| | |
| SEQ. ID No. 25: | Coli2Probe_b |
| | |
| SEQ. ID No. 26: | Coli2b_rw |
| SEQ. ID No. 27: | Coli2c_rw |
| SEQ. ID No. 28: | Coli2d_rw |
| SEQ. ID No. 29: | Coli2e_rw |

### Locus 4 (23S)

| | |
|---|---|
| SEQ. ID No. 30: | 125 Ecoli23Sprobe |
| SEQ. ID No. 31: | 69 Ecoli23S fw |
| SEQ. ID No. 32: | 164 Ecoli23s re |
| | |
| SEQ. ID No. 33: | Wolc Probe |
| SEQ. ID No. 34: | Wolc fw |

### D. Enterococcus faecalis

| | |
|---|---|
| SEQ. ID No. 35: | 405 Efaec16Sprobe |
| SEQ. ID No. 36: | 361 Efaec 16S fw |
| SEQ. ID No. 37: | 447 Efaec 16S re |
| | |
| SEQ. ID No. 38: | Efaec16Sprobe_1 |
| SEQ. ID No. 39: | Efaec16s fw_1 |

## Patentansprüche

1. Verfahren zur Ermittlung von Organismen in Wasser, insbesondere Trinkwasser, umfassend die Schritte:
a. Filtration einer Wasserprobe durch einen Filter;
b. Gewinnung von Organismenzellen aus dem Filter mit einem Lösungsmittel;
c. Lyse der Organismenzellen;
d. Bestimmung und/oder Quantifizierung der Organismen, insbesondere mittels RT-qPCR,
**dadurch gekennzeichnet, dass**
das Lösungsmittel und die Kontaktzeit und -bedingungen derart gewählt sind, dass der Filter in dem Lösungsmittel im Wesentlich aufgelöst wird und die Organismenzellen im Wesentlichen intakt bleiben.

2. Verfahren gemäss Anspruch 1, wobei der Filter mit einem polaren aprotischen Lösungsmittel im Wesentlichen aufgelöst wird.

3. Verfahren gemäss einem der Ansprüche 1 oder 2, wobei die Wasserprobe durch einen Celluloseester-Filter filtriert wird, der Celluloseester-Filter in einem polaren aprotischen Lösungsmittel im Wesentlichen aufgelöst wird und die Organismenzellen im Wesentlichen intakt aus dem polaren aprotischen Lösungsmittel gewonnen werden.

4. Verfahren gemäss einem der Ansprüche 1 oder 2, wobei die Wasserprobe durch einen Celluloseacetat Filter filtriert wird, der Filter mit Dimethylsulfoxid im Wesentlichen aufgelöst wird und die Organismenzellen im Wesentlichen intakt aus dem Dimethylsulfoxid gewonnen werden.

5. Set zur Ermittlung von Organismen in Wasser, insbesondere Trinkwasser, wobei das Set mindestens ein Lösungsmittel und mindestens einen Filter enthält, **dadurch gekennzeichnet, dass** das mindestens eine Lösungsmittel den Filter im Wesentlichen aufzulösen vermag und Organismenzellen im Wesentlichen intakt lässt.

6. Set zur Ermittlung von Organismen in Wasser gemäss Anspruch 5, weiter enthaltend eine Spritze zur Entnahme einer Wasserprobe des zu untersuchenden Trinkwassers.

7. Set zur Ermittlung von Organismen in Wasser gemäss Anspruch 6, weiter enthaltend ein Filtergehäuse zur Befestigung an die Spritze.

8. Set zur Ermittlung von Organismen in Wasser gemäss einem der Ansprüche 5 bis 7, wobei der Filter im Wesentlichen aus mindestens einem Celluloseester oder Celluloseacetat besteht und das Lösungsmittel mindestens ein polares aprotisches Lösungsmittel umfasst.

9. Set zur Ermittlung von Organismen in Wasser gemäss einem der Ansprüche 5 bis 8, wobei das Lösungsmittel mindestens ein polares aprotisches Lösungsmittel umfasst, ausgewählt aus der Gruppe bestehend aus: Ketonen, Lactonen, Nitrilen, Nitroverbindungen, tertiären Carbonsäureamiden, Harnstoffderivaten, Sulfoxiden, Sulfonen und Kohlensäureestern, sowie Mischungen davon.

10. Set zur Ermittlung von Organismen in Wasser gemäss einem der Ansprüche 5 bis 9, wobei das Lösungsmittel mindestens ein Lösungsmittel umfasst, ausgewählt aus der Gruppe bestehend aus:
Aceton, 4-Butyrolacton, Acetonitril, Nitromethan, Dimethylformamid, Tetramethylharnstoff, Dimethylpropylen-harnstoff (DMPU), Sulfolan, Dimethylcarbonat, Ethylencarbonat, Dimethylsulfoxid (DMSO), sowie Mischungen davon.

11. Set zur Ermittlung von Organismen in Wasser gemäss einem der Ansprüche 5 bis 10, wobei das Set weiterhin mindestens ein forward/reverse Primerpaar ausgewählt aus den Primern der folgenden Gruppe enthält: SEQ ID No. 2, 3, 4, 6, 7, 8, 9, 10, 11, 12, 15, 16, 18, 19, 21, 22, 23, 24, 26, 27, 28, 29, 31, 32, 34, 36, 37, 39.

12. Set zur Ermittlung von Organismen in Wasser gemäss einem der Ansprüche 5 bis 11, wobei das Set weiterhin mindestens eine Sonde ausgewählt aus der folgenden Gruppe enthält: SEQ ID No. 1, 5, 13, 14, 17, 20, 25, 30, 33, 35, 38, wobei die Sonde(n) an ihrem einen Ende, vorzugsweise am 3'-Ende, mit einem Fluoreszenzquencher, vorzugsweise BHQ1 oder TAMRA, modifiziert ist/sind, und an ihrem anderen Ende, vorzugsweise am 5'-Ende, mit einem Fluoreszenzfarbstoff, vorzugsweise FAM oder Yakima yellow, modifiziert ist/sind.

13. Forward/reverse PCR-Primerpaar, wobei die forward- und reverse Primer ausgewählt sind aus der Gruppe bestehend aus: SEQ ID No. 2, 3, 4, 6, 7, 8, 9, 10, 11, 12, 15, 16, 18, 19, 21, 22, 23, 24, 26, 27, 28, 29, 31, 32, 34, 36, 37, 39; vorzugsweise PCR Primerpaare SEQ ID No. 2 und 3, SEQ ID No. 2 und 4, SEQ ID No. 6 und 7.

14. PCR-Fluoreszenzsonde, ausgewählt aus der Gruppe bestehend aus: SEQ ID No. 1, 5, 13, 14, 17, 20, 25, 30, 33, 35, 38, wobei die Sonde(n) an ihrem einen Ende, vorzugsweise am 3'-Ende, mit einem Fluoreszenzquencher, vorzugsweise BHQ1 oder TAMRA, modifiziert ist/sind, und an ihrem anderen Ende, vorzugsweise am 5'-Ende, mit einem Fluoreszenzfarbstoff, vorzugsweise FAM oder Yakima yellow, modifiziert ist/sind.

15. Primer-/Sondenkit für die PCR, umfassend wenigstens ein Primerpaar gemäss Anspruch 13 und wenigstens eine Sonde gemäss Anspruch 14.


SEQUENCE LISTING

<110>  IMETH AG

<120>  Verfahren zur Ermittlung von Organismen in Wasser

<130>  PIMET001EP

<160>  39

<170>  PatentIn version 3.3

<210>  1
<211>  29
<212>  DNA
<213>  Escherichia coli

<400>  1
cctttgctca ttgacgttac ccgcagaag                                        29

<210>  2
<211>  20
<212>  DNA
<213>  Escherichia coli

<400>  2
cggggaggaa gggagtaaag                                                  20

<210>  3
<211>  22
<212>  DNA
<213>  Escherichia coli

<400>  3
caagcttgcc agtatcagat gc                                               22

<210>  4
<211>  22
<212>  DNA
<213>  Escherichia coli

<400>  4
gactcaagct tgccagtatc ag                                               22

<210>  5
<211>  27
<212>  DNA
<213>  Escherichia coli

<400>  5
cgcgtgagtg aagaaggttt tcggatc                                          27

<210>  6
<211>  20
<212>  DNA
<213>  Enterococcus faecalis

<400>  6
ggaatcttcg gcaatggacg                                                  20

<210>  7
<211>  25

<212>  DNA
<213>  Enterococcus faecalis

<400>  7
tcagttacta acgtccttgt tcttc                                          25


<210>  8
<211>  20
<212>  DNA
<213>  Escherichia coli

<400>  8
ctcaacctgg gaactgcatc                                                20


<210>  9
<211>  20
<212>  DNA
<213>  Escherichia coli

<400>  9
caacctggga actgcatctg                                                20


<210>  10
<211>  18
<212>  DNA
<213>  Escherichia coli

<400>  10
gaaatccccg ggctcaac                                                  18


<210>  11
<211>  22
<212>  DNA
<213>  Escherichia coli

<400>  11
gtattcctcc agatctctac gc                                             22


<210>  12
<211>  21
<212>  DNA
<213>  Escherichia coli

<400>  12
ctctacgcat ttcaccgcta c                                              21


<210>  13
<211>  25
<212>  DNA
<213>  Escherichia coli

<400>  13
ctggcaagct tgagtctcgt agagg                                          25


<210>  14
<211>  27
<212>  DNA
<213>  Escherichia coli

<400>  14
cggggaggaa gggagtaaag ttaatac                                        27

<210> 15
<211> 21
<212> DNA
<213> Escherichia coli

<400> 15
gtgtatgaag aaggccttcg g 21

<210> 16
<211> 20
<212> DNA
<213> Escherichia coli

<400> 16
cttcttctgc gggtaacgtc 20

<210> 17
<211> 28
<212> DNA
<213> Escherichia coli

<400> 17
cggggaggaa gggagtaaag ttaatacc 28

<210> 18
<211> 20
<212> DNA
<213> Escherichia coli

<400> 18
gccttcgggt tgtaaagtac 20

<210> 19
<211> 18
<212> DNA
<213> Escherichia coli

<400> 19
cttctgcggg taacgtca 18

<210> 20
<211> 31
<212> DNA
<213> Escherichia coli

<400> 20
cagcggggag gaagggagta aagttaatac c 31

<210> 21
<211> 24
<212> DNA
<213> Escherichia coli

<400> 21
gaaggccttc gggttgtaaa gtac 24

<210> 22
<211> 21
<212> DNA

<213>  Escherichia coli

<400>  22
gtgtatgaag aaggccttcg g                                                21

<210>  23
<211>  20
<212>  DNA
<213>  Escherichia coli

<400>  23
cttcttctgc gggtaacgtc                                                  20

<210>  24
<211>  20
<212>  DNA
<213>  Escherichia coli

<400>  24
cggggaggaa gggagtaaag                                                  20

<210>  25
<211>  29
<212>  DNA
<213>  Escherichia coli

<400>  25
cctttgctca ttgacgttac ccgcagaag                                        29

<210>  26
<211>  23
<212>  DNA
<213>  Escherichia coli

<400>  26
catctgactt aacaaaccgc ctg                                              23

<210>  27
<211>  22
<212>  DNA
<213>  Escherichia coli

<400>  27
cacatctgac ttaacaaacc gc                                               22

<210>  28
<211>  22
<212>  DNA
<213>  Escherichia coli

<400>  28
caagcttgcc agtatcagat gc                                               22

<210>  29
<211>  22
<212>  DNA
<213>  Escherichia coli

<400>  29
gactcaagct tgccagtatc ag                                               22

<210>  30
<211>  25
<212>  DNA
<213>  Escherichia coli

<400>  30
aaacccagtg tgattcgtca cacta                                          25


<210>  31
<211>  20
<212>  DNA
<213>  Escherichia coli

<400>  31
cgataagcgt cggtaaggtg                                                20


<210>  32
<211>  21
<212>  DNA
<213>  Escherichia coli

<400>  32
ggttcgcctc attaacctat g                                              21


<210>  33
<211>  28
<212>  DNA
<213>  Escherichia coli

<400>  33
cgaatgggga aacccagtgt gattcgtc                                       28


<210>  34
<211>  20
<212>  DNA
<213>  Escherichia coli

<400>  34
atgaaccgtt ataaccggcg                                                20


<210>  35
<211>  26
<212>  DNA
<213>  Enterococcus faecalis

<400>  35
tgagtgaaga aggttttcgg atcgta                                         26


<210>  36
<211>  19
<212>  DNA
<213>  Enterococcus faecalis

<400>  36
ggaatcttcg gcaatggac                                                 19


<210>  37
<211>  25
<212>  DNA
<213>  Enterococcus faecalis

<400>  37
tcagttacta acgtccttgt tcttc                                              25


<210>  38
<211>  27
<212>  DNA
<213>  Enterococcus faecalis

<400>  38
cgcgtgagtg aagaaggttt tcggatc                                            27


<210>  39
<211>  20
<212>  DNA
<213>  Enterococcus faecalis

<400>  39
ggaatcttcg gcaatggacg                                                    20